# EUROPEAN PATENT SPECIFICATION

(11) **EP 3 760 728 B1**
(45) Date of publication and mention of the grant of the patent: **29.11.2023**
(21) Application number: 18907612.8
(22) Date of filing: 21.12.2018
(51) Int. Cl.: C12Q 1/02, G01N 33/50, C12M 1/34, C12N 5/0793, C12N 5/10

(54) **EVALUATION OF CELLS USING DIRECTIONAL MOVEMENT ABILITY.**
BEWERTUNG VON ZELLEN ANHAND DER RICHTUNGSBEWEGUNGSFÄHIGKEIT.
ÉVALUATION DES CELLULES À L'AIDE DE LA CAPACITÉ DE MOUVEMENT DIRECTIONNEL.

(30) Priority: 28.02.2018 JP 2018035758
(43) Date of publication of application: 06.01.2021
(73) Proprietor: National University Corporation Tokai National Higher Education and Research System, Nagoya-shi Aichi 464-8601 (JP)
(72) Inventor: OZAKI, Norio, Nagoya-shi, Aichi 464-8601 (JP); ARIOKA, Yuko, Nagoya-shi, Aichi 464-8601 (JP); HIGASHIJIMA, Emiko, Nagoya-shi, Aichi 464-8601 (JP); MORI, Daisuke, Nagoya-shi, Aichi 464-8601 (JP); KUSHIMA, Itaru, Nagoya-shi, Aichi 464-8601 (JP)
(74) Representative: Jaekel, Robert Sebastian
(86) International application number: PCT/JP2018/047146
(87) International publication number: WO 2019/167398

(56) References cited:
- WO-A1-2015/042436
- WO-A1-2016/208755
- WO-A1-2017/009766
- WO-A1-2017/035315
- JP-A- 2010 119 314
- JP-A- 2011 004 608
- JP-A- 2013 039 113
- JP-A- 2014 179 061
- US-A1- 2014 377 831
- PETRIE RYAN J. ET AL: "Random versus directionally persistent cell migration", NAT REV MOL CELL BIOL., vol. 10, no. 8, 1 August 2009 (2009-08-01) , pages 538-549, XP55840179, London ISSN: 1471-0072, DOI: 10.1038/nrm2729 Retrieved from the Internet: URL:https://www.ncbi.nlm.nih.gov/pmc/artic les/PMC2752299/pdf/nihms144902.pdf>
- LOOSLEY ALEX J. ET AL: "Describing Directional Cell Migration with a Characteristic Directionality Time", PLOS ONE, vol. 10, no. 5, 20 May 2015 (2015-05-20), page e0127425, XP55840187, DOI: 10.1371/journal.pone.0127425 Retrieved from the Internet: URL:https://storage.googleapis.com/plos-co rpus-prod/10.1371/journal.pone.0127425/1/p one.0127425.pdf?X-Goog-Algorithm=GOOG4-RSA -SHA256&X-Goog-Credential=wombat-sa@plos-p rod.iam.gserviceaccount.com/20210913/auto/ storage/goog4_request&X-Goog-Date=20210913 T081727Z&X-Goog-Expires=86400&X-Goog-Signe dHeaders=h>
- WEIGER MICHAEL C. ET AL: "Real-Time Motion Analysis Reveals Cell Directionality as an Indicator of Breast Cancer Progression", PLOS ONE, vol. 8, no. 3, 19 March 2013 (2013-03-19), page e58859, XP55840243, DOI: 10.1371/journal.pone.0058859 Retrieved from the Internet: URL:https://www.ncbi.nlm.nih.gov/pmc/artic les/PMC3602596/pdf/pone.0058859.pdf>
- ARIOKA YUKO ET AL: "Single-cell trajectory analysis of human homogenous neurons carrying a rare RELN variant", TRANSLATIONAL PSYCHIATRY, vol. 8, no. 1, 1 December 2018 (2018-12-01), page 129, XP55840335, DOI: 10.1038/s41398-018-0177-8 Retrieved from the Internet: URL:https://www.nature.com/articles/s41398 -018-0177-8.pdf>
- ARIOKA YUKO ET AL: "Cell body shape and directional movement stability in human-induced pluripotent stem cell-derived dopaminergic neurons", SCIENTIFIC REPORTS, vol. 10, no. 1, 2 April 2020 (2020-04-02), pages 1-9, XP55840327, US ISSN: 2045-2322, DOI: 10.1038/s41598-020-62598-4 Retrieved from the Internet: URL:https://www.nature.com/articles/s41598 -020-62598-4.pdf>
- TIENG V. et al.: "Engineering of Midbrain Organoids Containing Long-Lived Dopaminergic Neurons", Stem Cells and Development, vol. 23, no. 13, 2014, pages 1535-1547, XP055423743, DOI: doi:10.1089/scd.2013.0442

## Description

### [Technical Field]

The present invention relates to a cell-based assay system. More specifically, the present invention relates to a drug evaluation method using a cell-based assay system to obtain a movement pattern defined by directional movement ability of individual cells constituting a cell population of neurons derived from induced pluripotent stem cells as an index and its application

### [Background Art]

As a pathological hypothesis of schizophrenia (SCZ) and autism spectrum disorder (ASD), it is thought that neurodevelopmental disorder plays an important role. Not only in ASD whose characteristics have already become apparent from the developmental stage, but also in SCZ, it has been reported that there are some cognitive dysfunctions and neurophysiological or neuroimaging changes before the onset or the manifestation of obvious psychiatric symptoms (NPL 1), and that, according to studies using the postmortem brains of patients with SCZ and ASD, a failure in the layer structure of brain is seen in these patients (NPL 2). These reports suggest that neurodevelopmental disorders that begin in the fetal period are causes of the onset of SCZ and ASD, but details of the pathologic conditions in the brain of patients with SCZ and ASD are unknown.

SCZ and ASD genome analysis studies have identified a plurality of mutations in genes involved in neurodevelopment in both diseases, one of which is a mutation in RELN (for example, see NPL 3). The protein Reelin encoded by RELN is a large secreted protein and is essential for the formation of the layer structure of the developing brain. In humans, a RELN homozygous deletion mutation causes lissencephaly with developmental delay, and a decrease in Reelin has been reported to be associated with the onset of neurodevelopmental disorders (NPL 4). Similarly, in reeler mice, which are Reln mutant mice, a disorder of brain layer structure and an abnormal behavior have been reported (for example, see NPL 5). These reports suggest that even in humans, a decrease in Reelin in the brain may cause unstable neuronal migration, which is expected to cause neurodevelopmental disorders related to SCZ and ASD.

### [Citation List]

### [Non Patent Literature]

[NPL 1] Schizophrenia bulletin, 29(4):653-669, 2003
[NPL 2] Dialogues Clin Neurosci, 2(4):349-357, 2000
[NPL 3] Mol Psychiatry 22(3) 430-440, 2017
[NPL 4] Nat Genet 26(1):93-96, 2000
[NPL 5] Nature Rev Neurosci, 4(6):496-505, 2003

Nat Rev Mol Cell Biol., 10 (8):538-549, 2009 describes generally directional migration as an important component of cell motility and distance as one measure of directionality of movement.

JP 2013 039113 A describes a method of cell quality control based on cell displacement.

JP 2014 179061 A describes a system for quality control of nerve cells based on movement analysis.

### [Summary of Invention]

### [Technical Problem]

The present invention relates to the embodiments as characterized in the claims. There are several subtypes of neurons that express Reelin. Among them, tyrosine hydroxylase (TH)-positive dopamine neurons have been reported, in researches using mice, to express Reelin only in a limited period before and after birth. Although Reelin is expressed only in such a limited period, abnormalities in dopamine neurons have been confirmed in Rein-mutated reeler mice. On the other hand, the presence of many reports that the dopamine system is involved in the pathologic conditions of SCZ and ASD suggests the possibility that, through investigation of the relationship between dopamine neurons and Reelin, clues to elucidate the mechanism for onset of SCZ and ASD may be obtained.

The present inventors have advanced researches, focusing on Reelin, in order to elucidate the onset mechanism of SCZ and ASD and to establish a treatment method therefor. As one of the research results, the present inventors have succeeded in establishing a method for specifically and efficiently preparing dopamine neurons from iPS cells (Japanese Patent Application No. 2017-82600).

Under the above background, an object of the present invention is to provide a novel evaluation system that is particularly useful in researches on the onset mechanism and pathologic conditions of a central nervous system disease (for example, a mental disease), development of a therapeutic method, or the like.

### [Solution to Problem]

As described above, the present inventors have established a new method for preparing dopamine neurons. In this method, neurospheres (neuron aggregates) are formed in the process of differentiation induction, and, when the neurospheres are cultured under conditions of inducing differentiation into neurons, cells (dopamine neurons) move to the surroundings. As a result of repeated studies focusing on the movement distance and direction of each of the individual cells that moved, it has been found that the movement pattern of healthy subject-derived cells is characteristic (the cells show a specific movement pattern) and that RELN-deficient mental disease patient-derived cells show a movement pattern different from that of the healthy subject-derived cells. This finding means that the movement pattern defined by the movement characteristics (in particular, movement direction) of the individual cells constituting a cell population reflects the disease state, and indicates that an evaluation system using this movement pattern as an index is useful, for example, not only in basic researches (for example, elucidation of the disease mechanism), but also in understanding pathologic conditions and developing therapeutic drugs. When used in the development of therapeutic drugs, normalization of the movement pattern demonstrates efficacy, and abnormalization of the movement pattern demonstrates toxicity. For example, when RELN-deficient mental disease patient-derived cells are used, the normalization of the movement pattern can indicate the efficacy of rescuing endogenous Reelin. Here, in view of the fact that the cell movement ability is important for normal formation and regeneration processes of various tissues and organs in addition to the central nervous system, the coverage (target cells, uses/applications, etc.) of the above evaluation system focusing on the movement characteristics of cells is broad, i.e., the evaluation system can be widely used as an evaluation system for efficacy or toxicity, and its practical value and significance are extremely high. Further studies by the present inventors have also provided various findings useful and important for putting the above evaluation system into practical use.

### [Brief Description of Drawings]

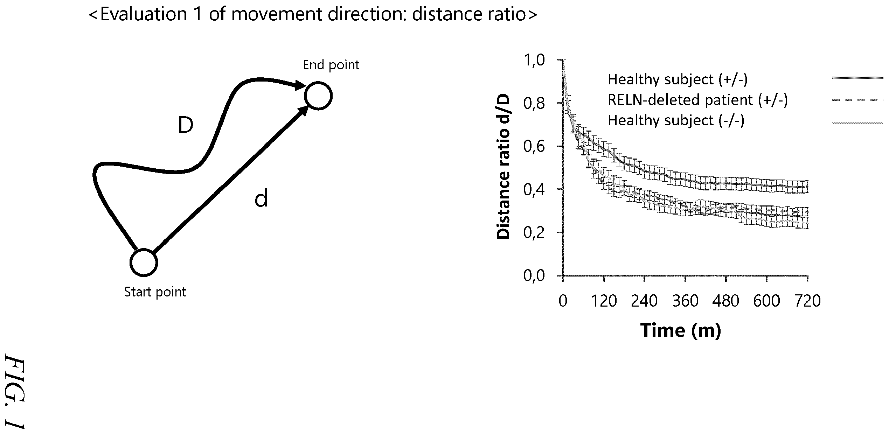

[Fig. 1] A method for evaluating a movement direction using a distance ratio (left) and an example of evaluation results (measurement over time) by the evaluation method (right). When a cell moved during a time t, an actual total movement distance was defined as D(t), and a linear distance between two points, i.e., a start point and an end point was defined as d(t). The movement direction was evaluated by a d(t)/D(t) value. The d(t)/D(t) value is 1 when the cell has moved in a completely linear manner, and becomes smaller as the cell meanders. Healthy subject (+/+): neurons derived from iPS cells of healthy subjects; RELN-deleted patient (+/-): neurons derived from RELN-deleted patients; and healthy subject (-/-): neurons derived from strains of healthy subjects RELN-homozygous-deleted by genome editing.
[Fig. 2] A method for evaluating the movement direction using a vector and an angle (left) and an example of evaluation results by the evaluation method (right). When a cell moved within a certain time, an average movement vector from the movement start point to the movement end point was calculated. An angle (θ) between a position vector at a certain time point and the average movement vector, of the cell on the way of movement, was determined, and the distribution of θ was represented by a histogram and a circular histogram (or radar plot) for each cell population.
[Fig. 3] Evaluation results of a movement pattern of healthy subject iPS cell-derived neurons. CON1: Dopamine neurons induced from a healthy female; and CON2: Dopamine neurons induced from a healthy male.
[Fig. 4] Comparison between the movement pattern of RELN-deleted patient iPS cell-derived neurons (RELN-del1) and the movement pattern of healthy subject iPS cell-derived neurons (CON1).
[Fig. 5] Comparison between the movement pattern of RELN deletion and the movement pattern of healthy subjects.
[Fig. 6] Automatic detection results of the movement pattern. There was constructed a measurement system for automatically detecting/quantifying variations in movement of the individual cells.
[Fig. 7] Evaluation of the movement pattern using an automatic detection system. Comparison was made between the movement pattern of the healthy subject iPS cell-derived neurons (CON1) and the movement patterns of RELN-deleted groups (RELN-deleted patient iPS cell-derived neurons (RELN-del1) and RELN-homozygous-deleted isogenic iPS cell-derived neurons (CON1 (-/-)).

### [Description of Embodiments]

The present invention relates to the embodiments as characterized in the claims. The present invention provides a drug evaluation method used in the evaluation of the efficacy or toxicity of a test substance (hereinafter referred to also as "evaluation method of the present invention"). In the present specification, the term "drug evaluation" is used as a general term for evaluation of efficacy and toxicity. Therefore, the efficacy or toxicity of the test substance is evaluated in the present invention. The term "toxicity" should be interpreted in a broad sense, and includes, in addition to general toxicities (acute toxicity, subacute toxicity, and chronic toxicity), side effects, carcinogenicity, mutagenicity, and teratogenicity.

The evaluation method of the present invention is an evaluation system utilizing neurons derived from induced pluripotent stem cells, involving preparing a specific cell population, and evaluating the efficacy or toxicity of a test substance using, as an index, a movement pattern defined by directional movement ability of individual cells constituting the cell population (which is associated with the degree (level) of variations in movement direction of the cells and the movement velocity (speed)). The studies by the present inventors have revealed that when attention is paid to the variations in movement of the individual cells constituting the cell population, the "movement pattern" that represents the movement characteristics of the cell population can be defined, and that the "movement pattern" is correlated with the normality (abnormality, in other words) of the cell population and is useful for evaluation of efficacy or toxicity (see Examples below).

Typically, the evaluation method of the present invention includes the following steps (i) to (iii) of:
(i) preparing a population of cells exhibiting movement ability, starting culture in the presence of a test substance, and then measuring positions of the individual cells that have moved over time,
(ii) analyzing the directional movement ability of the respective cells from information on the measured positions, totalizing the analysis results, and determining a movement pattern of the cell population, and
(iii) determining efficacy or toxicity of the test substance based on the determined movement pattern.

In step (i), a population of cells exhibiting movement ability is prepared, and culture is started in the presence of a test substance. In the present invention, since the efficacy or toxicity of the test substance is evaluated using the movement pattern as an index, a cell population constituted by cells exhibiting directional movement ability is used. However, it is not essential that all the cells constituting the cell population exhibit directional movement ability. That is, the mixture of cells that do not exhibit the directional movement ability is not inhibited. The term "mixture (mix)" as used herein means that such cells are present in the cell population but are not major constituent cells. Therefore, the abundance (mixing rate) of such cells is, for example, 40% or less of the entire cell population, preferably 30% or less of the entire cell population, more preferably 20% or less of the entire cell population, still more preferably 10% of the entire cell population.

Cells that exert a predetermined movement ability in the process of normal generation or formation, or regeneration of structures such as tissues and organs of the living body (particularly, cells that move in a certain direction and serve as a constituent component of specific tissues or organs) correspond to the "cells exhibiting directional movement ability" and are suitable as cells constituting the cell population in the method of the present invention, wherein the cells are neurons derived from induced pluripotent stem cells. Examples of the cells exhibiting directional movement ability include neurons (dopamine neurons, glutamatergic neurons, serotonin neurons, and GABAergic neurons), cardiomyocytes, and hematopoietic cells (leucocytes).

The cell population may include two or more kinds of cells which are different in cell types. Further, it may include two or more kinds of cells which are different in degree of differentiation.

Normal cells or abnormal cells can be used as the cells constituting the cell population. The "normal cell" is a cell that has no abnormality in relation with the efficacy (improvement in cell movement disorder and therapeutic effect based thereon) or toxicity evaluated by the method of the present invention. Typical examples of the normal cells are cells derived from healthy subjects. For example, cells derived from persons not suffering from a disease against which the efficacy to be evaluated can exert a therapeutic effect, in other words, a disease of which the onset and progress are caused at least by cell movement disorder (hereinafter referred to as "target disease") can also be used as the normal cells. The "abnormal cell" is a cell that is in contrast to the normal cell, and refers to a cell that is no longer in its original state due to gene mutation, chromosomal abnormality, or the like. Examples of the abnormal cells are cells having a genetic abnormality characteristic of the target disease, and cells derived from patients suffering from the target disease correspond to the abnormal cells. Examples of the patient-derived cells include cells collected from patients or subcultured cells thereof, and differentiated cells obtained by inducing differentiation of patient-derived induced pluripotent stem (iPS) cells (iPS cells prepared using cells collected from patients). In addition to patient-derived cells, cells in which a genetic abnormality characteristic of the target disease is introduced (for example, differentiated cells obtained by introducing a gene mutation into undifferentiated cells such as iPS cells by gene manipulation such as genome editing, and then inducing differentiation of the cells) and cells obtained by introducing a gene mutation into differentiated cells can also be adopted as the abnormal cell. Examples of the target disease include neurodevelopmental disorders such as ASD and SCZ, and blood diseases developing leukocyte migration disorder, and examples of the genetic abnormality include deletion of Reelin gene, deletion of chromosome 3q29 region, and deletion of chromosome 22q11 region.

When patient-derived cells are used as the abnormal cells, the toxicity of therapeutic drugs or therapeutic drug candidates can also be evaluated (details will be described later), in addition to the evaluation of the efficacy for the purpose of screening (searching) for the therapeutic drug candidates. As described above, the method of the present invention has a unique characteristic that it can be used in both the efficacy evaluation system and the toxicity evaluation system by selecting the cells to be used. Details of each evaluation system will be described later.

In a preferred one embodiment, dopamine neurons prepared by the method including the following steps (1) to (3) are used as the cells in step (i).
(1) culturing pluripotent stem cells in the presence of a TGF-β family inhibitor, a GSK3β inhibitor, and a BMP inhibitor;
(2) suspension-culturing the cells obtained in step (1) in the presence of a TGF-β family inhibitor, a GSK3β inhibitor, FGF8, and a hedgehog signal agonist and under normal oxygen partial pressure to form a neurosphere; and
(3) collecting the cells constituting the neurosphere to induce differentiation thereof into dopamine neurons, or directly inducing the neurosphere into dopamine neurons.

### Step (1)

In step (1), pluripotent stem cells are used. The "pluripotent stem cell" refers to a cell having both the potential for differentiating into all cells constituting the body (pluripotency), and the potential for producing daughter cells having the same differentiation potency via cell division (self-replication competence). The pluripotency can be evaluated by transplanting cells of an evaluation subject into a nude mouse, and testing the presence or absence of formation of teratoma containing each cell of the three germ layers (ectoderm, mesoderm, and endoderm).

Examples of the pluripotent stem cells include induced pluripotent stem cells (iPS cells).. iPS cells are used. Preferably, pluripotent stem cells are cells of mammals (for example, primates such as humans or chimpanzees, and rodents such as mice or rats), and particularly preferably, pluripotent stem cells are human cells. Therefore, in a most preferable embodiment, human iPS cells are used as pluripotent stem cells.

The "induced pluripotent stem cell (iPS cell)" refers to a cell having pluripotency and self-replication competence, produced by reprogramming somatic cells (e.g., fibroblasts, skin cells, and lymphocytes), for example, through the introduction of initializing factors. iPS cells show characteristics similar to those of ES cells. Somatic cells used in the preparation of iPS cells are not particularly limited, and may be differentiated somatic cells or undifferentiated stem cells. iPS cells can be prepared by various methods reported so far. The application of iPS cell preparation methods which will be developed in the future is also contemplated. Examples of the cells available for preparing iPS cells (i.e. cells from which iPS cells are derived) include lymphocytes (T cells, B cells), fibroblasts, epithelial cells, endothelial cells, mucosal epithelial cells, mesenchymal cells, hematopoietic stem cells, adipose stem cells, dental pulp stem cells and neural stem cells.

The most fundamental technique of iPS cell preparation methods is to introduce four factors of Oct3/4, Sox2, Klf4, and c-Myc, which are transcription factors, into cells by using virus (Takahashi K, Yamanaka S: Cell 126 (4), 663-676, 2006; Takahashi, K, et al.: Cell 131 (5), 861-72, 2007). The establishment of human iPS cells by introduction of four factors of Oct4, Sox2, Lin28, and Nonog has been reported (Yu J, et al.: Science 318 (5858), 1917-1920, 2007). The establishment of iPS cells by introduction of three factors other than c-Myc (Nakagawa M, et al.: Nat. Biotechnol. 26 (1), 101-106, 2008), two factors of Oct3/4 and Klf4 (Kim JB, et al.: Nature 454 (7204), 646-650, 2008), or only Oct3/4 (Kim JB, et al.: Cell 136 (3), 411-419, 2009) has also been reported. Also, a technique for introducing a protein, which is an expression product of a gene, into cells (Zhou H, Wu S, Joo JY, et al.: Cell Stem Cell 4, 381-384, 2009; Kim D, Kim CH, Moon JI, et al: Cell Stem Cell 4, 472-476, 2009) has also been reported. On the other hand, it has been reported that it is possible to improve the preparation efficiency and reduce the factors to be introduced, by using, for example, an inhibitor BIX-01294 against histone methyltransferase G9a, a histone deacetylase inhibitor valproic acid (VPA), or BayK8644 (Huangfu D, et al.: Nat. Biotechnol. 26 (7), 795-797, 2008; Huangfu D, et al.: Nat. Biotechnol. 26 (11), 1269-1275, 2008; Silva J, et al.: PLoS. Biol. 6 (10), e 253, 2008). Studies have also been advanced on gene transfer methods, and techniques utilizing not only retroviruses, but also lentiviruses (Yu J, et al.: Science 318 (5858), 1917-1920, 2007), adenoviruses (Stadtfeld M, et al.: Science 322 (5903), 945-949, 2008), plasmids (Okita K, et al.: Science 322 (5903), 949-953, 2008), transposon vectors (Woltjen K, Michael IP, Mohseni P, et al.: Nature 458, 766-770, 2009; Kaji K, Norrby K, Pac a A, et al.: Nature 458, 771-775, 2009; Yusa K, Rad R, Takeda J, et al.: Nat Methods 6, 363-369, 2009), or episomal vectors (Yu J, Hu K, Smuga-Otto K, Tian S, et al: Science 324, 797-801, 2009) for gene transfer have been developed.

Cells in which transformation into iPS cells, i.e., initialization (reprogramming) has occurred can be selected by using, as an indicator, the expression of pluripotent stem cell markers (undifferentiated markers) such as Fbxo15, Nanog, Oct/4, Fgf-4, Esg-1, and Cript.

iPS cells can also be provided from, for example, the National University Corporation, Kyoto University or the Independent Administrative Institution, RIKEN BioResource Center.

Pluripotent stem cells can be maintained in vitro by known methods. For example, when it is desired to provide highly safe cells (e.g. in a case where clinical application is considered), pluripotent stem cells are preferably maintained by serum-free culture using a serum alternative or by feeder-free cell culture. If a serum is used (or used in combination), autologous serum (i.e., recipient's serum) is preferably used. A serum alternative can be prepared by known methods (see, for example, WO 98/30679). Commercially available serum alternatives can also be used. Examples of the commercially available serum alternatives include KSR (manufactured by Invitrogen), Chemically-defined Lipid concentrated (manufactured by Gibco), and Glutamax (manufactured by Gibco).

In step (1), the pluripotent stem cells prepared as described above are cultured in the presence of a TGF-β family inhibitor, a GSK3β inhibitor, and a BMP inhibitor. That is, the pluripotent stem cells are cultured using a medium to which a TGF-β family inhibitor, a GSK3β inhibitor, and a BMP inhibitor are added. Step (1) aims to enhance the neuronal differentiation potency of the pluripotent stem cells.

The medium to be used can be prepared using a medium used for culturing mammalian cells as a basal medium. As the basal medium, for example, a BME medium, a BGJb medium, a CMRL 1066 medium, a Glasgow MEM medium, an Improved MEM Zinc Option medium, an IMDM medium, a Medium 199 medium, an Eagle MEM medium, an αMEM medium, a DMEM medium, a Ham's medium, a Ham's F-12 medium, a RPMI 1640 medium, a Fischer's medium, a Neurobasal medium, and a mixed medium thereof can be used, and the basal medium is not particularly limited as long as the it can be used for culturing mammalian cells. For example, a mixed medium of IMDM medium and Ham's F-12 medium can be used. The mixing ratio is, for example, IMDM: Ham's F-12 = 0.8 to 1.2:1.2 to 0.8 in a volume ratio.

A TGF-β family inhibitor, a GSK3β inhibitor, and a BMP inhibitor are added to the medium. The TGF-β family inhibitor is a substance that inhibits TGF-β signaling through binding between TGF-β and a TGF-β receptor. The TGF-β inhibitor includes proteinaceous inhibitors and small molecule inhibitors. Examples of such proteinaceous inhibitors are anti-TGF-β neutralizing antibodies and anti-TGF-β receptor neutralizing antibodies. Examples of such small molecule inhibitors are SB431542 (4-[4-(1,3-benzodioxol-5-yl)-5-(2-pyridinyl)-1H-imidazol-2-yl]-benzamide or its hydrate), SB202190 (4-(4-fluorophenyl)-2-(4-hydroxyphenyl)-5-(4-pyridyl)-1H-imidazole), SB505124 (GlaxoSmithKline), NPC30345, SD093, SD908, SD208 (Scios), LY2109761, LY364947, and LY580276 (Lilly Research Laboratories). Preferably, SB431542 is used. The concentration of the TGF-β family inhibitor (amount to be added to the medium) is not particularly limited as long as the purpose of enhancing the neuronal differentiation potency of the pluripotent stem cells is achieved. When SB431542 is taken as an example, the concentration thereof is, for example, 0.5 µM to 20 µM, preferably 1 µM to 10 µM. The optimum concentration can be set through preliminary experiments. Instead of keeping the TGF-β family inhibitor concentration constant throughout the entire culture period, changes in TGF-β family inhibitor concentration, e.g., a stepwise increase in TGF-β family inhibitor concentration, may be provided.

Examples of usable GSK3β inhibitors include CHIR99021 (6-[[2-[[4-(2,4-dichlorophenyl)-5-(4-methyl-1H-imidazol-2-yl)-2-pyrimidinyl]amino]ethyl]amino]nicotinonitrile), SB-415286(3-[(3-chloro-4-hydroxyphenyl)amino]-4-(2-nitrophenyl)-1H-pyrrole-2,5-dione), SB-2167, Indirubin-3'-Monoxime, Kenpaullone, and BIO (6-bromoindirubin-3'-oxime). Preferably, CHIR99021 is used. The concentration of the GSK3β inhibitor (amount to be added to the medium) is not particularly limited as long as the purpose of enhancing the neuronal differentiation potency of the pluripotent stem cells is achieved. When CHIR99021 is taken as an example, the concentration thereof is, for example, 0.5 µM to 20 µM, preferably 1 µM to 10 µM. The optimum concentration can be set through preliminary experiments. Instead of keeping the GSK3β inhibitor concentration constant throughout the entire culture period, changes in GSK3β inhibitor concentration, e.g., a stepwise increase in GSK3β inhibitor concentration, may be provided.

The BMP inhibitor is a substance that inhibits BMP signaling through binding between BMP (bone morphogenetic protein) and a BMP receptor (type I or type II). The BMP inhibitor includes proteinaceous inhibitors and small molecule inhibitors. Examples of such proteinaceous inhibitors include natural inhibitors such as Noggin, chordin and follistatin. Examples of such small molecule inhibitors include Dorsomorphin (6-[4-(2-piperidin-1-ylethoxy)phenyl]-3-pyridin-4-ylpyrazolo[1,5-a]pyrimidine) and its derivatives, LDN-193189 (4-(6-(4-piperazin-1-yl)phenyl) pyrazolo[1,5-a]pyrimidin-3-yl) quinoline) and its derivatives. These compounds are commercially available (e.g., available from Sigma-Aldrich and Stemgent) and are readily available. Preferably, Dorsomorphin is used. The concentration of the BMP inhibitor (amount to be added to the medium) is not particularly limited as long as the purpose of enhancing the neuronal differentiation potency of the pluripotent stem cells is achieved. When Dorsomorphin is taken as an example, the concentration thereof is, for example, 0.5 µM to 20 µM, preferably 1 µM to 10 µM. The optimum concentration can be set through preliminary experiments. Instead of keeping the BMP inhibitor concentration constant throughout the entire culture period, changes in BMP inhibitor concentration, e.g., a stepwise increase in BMP inhibitor concentration, may be provided.

As necessary, the medium can contain other components. Examples of the components to be added include insulin, an iron source (e.g., transferrin), a mineral (e.g., sodium selenate), a saccharide (e.g., glucose), an organic acid (e.g., pyruvic acid or lactic acid), a serum protein (e.g., albumin), an amino acid (e.g., L-glutamine), a reducing agent (e.g., 2-mercaptoethanol), a vitamin (e.g., ascorbic acid or d-biotin), an antibiotic (e.g., streptomycin, penicillin or gentamicin), and a buffer (e.g., HEPES).

Pluripotent stem cells are usually subjected to adherent culture. The adherent culture is in contrast to suspension culture, and is typically two-dimensional culture (plane culture) under adherent conditions. However, Matrigel^{™} (BD) or the like may be used for three-dimensional culture. For example, dishes, Petri dishes, tissue culture dishes, multi-dishes, microplates, microwell plates, multi-plates, multi-well plates, chamber slides, laboratory dishes, and the like can be used for adherent culture. In order to enhance the adhesiveness of the cells to the culture surface, it is preferable to use a culture vessel coated with Matrigel^{™} (BD), poly-D-lysine, poly-L-lysine, collagen, gelatin, laminin, heparan sulfate proteoglycan, entactin, or a combination of two or more thereof.

The pluripotent stem cells can be cultured under either condition, i.e., either in the presence or absence of feeder cells. When it is desired to provide highly safe cells, e.g., when clinical application is taken into consideration, the pluripotent stem cells may be cultured in the absence of feeder cells (feeder cell-free culture). Examples of the feeder cells are MEFs (mouse embryonic fibroblasts), STO cells (mouse embryonic fibroblast cell line), and SNL cells (subclones of the STO cells).

Other culture conditions such as culture temperature, CO₂ concentration, and O₂ concentration can be set as appropriate. The culture temperature is, for example, about 30 to 40°C, preferably about 37°C. The CO₂ concentration is, for example, about 1 to 10%, preferably about 5%. Moreover, culture may be performed under normal oxygen partial pressure. The oxygen concentration in the case of the condition "under normal oxygen partial pressure" is typically about 18% to about 22%, though it may vary depending on other conditions (humidity, CO₂ concentration, and the like). The details of the condition "under normal oxygen partial pressure" will be described later.

The period (culture period) of step (1) is 4 days or longer, specifically, for example, 4 days to 20 days, preferably 6 days to 14 days. If the culture period is too short, the neurosphere formation ability will be reduced.

The cells may be subjected to subculture as necessary. For example, the cells are collected at a stage where they are brought in a subconfluent or confluent state, a part of the cells is seeded in another culture vessel, and culture is continued. A cell dissociation solution or the like may be used for cell collection. As the cell dissociation solution, for example, proteases such as EDTA-trypsin, collagenase IV, and metalloprotease can be used alone or in an appropriate combination. Cell dissociation solutions with low cell toxicity are preferred. As such cell dissociation solutions, commercially available products such as DISPASE (EIDIA Co., Ltd.), TrypLE (Invitrogen), and Accutase (MILLIPORE) are available. The collected cells may be subjected to subculture after treatment with a cell strainer or the like so as to arrive at a dispersed (discrete) state.

As a result of step (1), the neuronal differentiation potency of the pluripotent stem cells is enhanced. Increased neuronal differentiation potency can be confirmed by using as an index an increase in expression of nervous system markers (Sox2, nestin, Sox1, and the like) as compared with that before the start of step (1). Moreover, the expression of an undifferentiation marker may be utilized for evaluation of increased neuronal differentiation potency.

Usually, the cells after step (1) are once collected and then the process proceeds to the next culture (step (2)). The collection operation can be performed in the same manner as the collection operation during subculture.

### Step (2)

In this step, the cells obtained in step (1) are cultured in suspension in the presence of a TGF-β family inhibitor, a GSK3β inhibitor, FGF8, and a hedgehog signal agonist and under normal oxygen partial pressure to form a neurosphere. That is, the cells after step (1) are cultured using a medium to which a TGF-β family inhibitor, a GSK3β inhibitor, FGF8, and a hedgehog signal agonist are added and under the condition of normal oxygen partial pressure. Step (2) aims to induce differentiation along the neuron lineage. The features not specifically mentioned (usable basic medium, usable TGF-β family inhibitor, GSK3β inhibitor, other components that can be added to the medium, and the like) are the same as those in step (1), and the explanations thereof are omitted.

For example, flasks, tissue culture flasks, dishes, Petri dishes, tissue culture dishes, multi-dishes, microplates, microwell plates, micropores, multi-plates, multi-well plates, chamber slides, laboratory dishes, tubes, trays, culture bags, roller bottles, and the like can be used for suspension culture. In order to enable culture under non-adherent conditions, it is preferable to use a culture vessel having a non-cell-adherent culture surface. Examples of the culture vessel involved include culture vessels whose surfaces (culture surfaces) have been treated to be non-cell-adherent, and culture vessels whose surfaces (culture surfaces) have not undergone a treatment for improving the cell adhesiveness (for example, coating treatment with an extracellular matrix). It is only necessary to maintain the non-adherent state of the cells to the culture vessel in suspension culture. Static culture may be employed, or swirl culture or shaking culture may be employed.

Among the components added to the medium, the TGF-β family inhibitor and the GSK3β inhibitor are as described above. Also in this step, it is preferable to use SB431542 as the TGF-β family inhibitor and CHIR99021 as the GSK3β inhibitor. The concentration of the TGF-β family inhibitor in the medium when SB431542 is used is, for example, 0.5 µM to 20 µM, preferably 1 µM to 10 µM. Similarly, the concentration of the GSK3β inhibitor in the medium when CHIR99021 is used is, for example, 0.5 µM to 20 µM, preferably 1 µM to 10 µM.

FGF8 is a member of the fibroblast growth factor family. FGF8 is involved in the control of vertebrate brain formation and is required for regionalization to the midbrain. As long as the object of the present invention can be achieved, FGF8s derived from various mammals can be used. However, it is preferable to use FGF8 derived from the same origin (animal species) as that of the pluripotent stem cells to be used. Therefore, human FGF8 is preferably used when human pluripotent stem cells are used. The "human FGF8" means FGF8 having an amino acid sequence of FGF8 naturally expressed in the human body, and may be a recombinant. As a typical amino acid sequence of human FGF8, the NCBI accession number: NP_006110.1 (fibroblast growth factor 8 isoform B precursor [Homo sapiens].) can be exemplified. The concentration of FGF8 (amount to be added to the medium) is not particularly limited as long as the purpose of inducing differentiation along the neuronal lineage is achieved, but is, for example, 1 ng/ml to 5 µg/ml, preferably 10 to 500 ng/ml, more preferably 50 to 400 ng/ml. The optimum concentration can be set through preliminary experiments.

The hedgehog signal agonist is not particularly limited as long as it promotes a sonic hedgehog (SHH) signal. For example, purmorphamine (9-cyclohexyl-N-[4-(4-morpholinyl)phenyl]-2-(1-naphthalenyloxy)-9H-purin-6-amine) useful for inducing ventralization is preferably used. The concentration of purmorphamine (amount added to the medium) is not particularly limited as long as the purpose of inducing differentiation along the neuron lineage is achieved, but is, for example, 1 ng/ml to 5 µg/ml, preferably 10 to 500 ng/ml, more preferably 50 to 400 ng/ml. The optimum concentration can be set through preliminary experiments. SAG (N-methyl-N'-(3-pyridinylbenzyl)-N'-(3-chlorobenzo[b]thiophene-2-carbonyl)-1,4-diaminocyclohexane)can also be used as the hedgehog signal agonist. The concentration of SAG used (amount added to the medium) is not particularly limited as long as the purpose of inducing differentiation along the neuron lineage is achieved, but is, for example, 10 nM to 100 µM, preferably 100 nM to 10 µM, more preferably 100 nM to 2 µM. The optimum concentration can be set through preliminary experiments.

It is not essential that the concentrations of the respective components (TGF-β family inhibitor, GSK3β inhibitor, FGF8 and hedgehog signal agonist) be constant throughout the entire culture period, and the concentration(s) of a specific component (which may be two or more components) or all the components may change during the culture. For example, the addition of FGF8 and hedgehog signal agonist is started on the second to sixth days of step (2). According to this condition, rapid stimulation to cells can be relieved. Preferably, the addition of FGF8 and hedgehog signal agonist is started on the third to fifth days of step (2).

In order to promote differentiation induction along the neuron lineage, a medium to which a leukemia inhibitory factor (LIF) is also added is preferably used. As long as the object of the present invention can be achieved, LIFs derived from various mammals can be used. However, it is preferable to use LIF derived from the same origin (animal species) as that of the pluripotent stem cells to be used. Therefore, human LIF is preferably employed when human pluripotent stem cells are used. The concentration of the LIF is not particularly limited, but is, for example, 0.25 ng/ml to 1 µg/ml, preferably 1 ng/ml to 50 ng/ml, more preferably 5 ng/ml to 20 ng/ml. The optimum concentration can be set through preliminary experiments.

In order to promote differentiation induction along the neuronal cell lineage, a medium to which a bFGF (basic fibroblast growth factor) is also added is preferably used. The bFGF is also called FGF2. As long as the object of the present invention can be achieved, bFGFs derived from various mammals can be used. However, it is preferable to use bFGF derived from the same origin (animal species) as that of the pluripotent stem cells to be used. Therefore, human bFGF is preferably employed when human pluripotent stem cells are used. The "human FGF2" means FGF2 having an amino acid sequence of FGF2 naturally expressed in the human body. As a representative amino acid sequence of human FGF2, the NCBI accession number: NP_001997.5 (fibroblast growth factor 2 [Homo sapiens]) can be exemplified. The concentration of the bFGF is not particularly limited, but is, for example, 0.25 ng/ml to 1 µg/ml, preferably 1 ng/ml to 50 ng/ml, more preferably 3 ng/ml to 30 ng/ml. The optimum concentration can be set through preliminary experiments.

In order to suppress cell death, a medium to which a ROCK inhibitor (Rho-associated coiled-coil forming kinase/Rho-binding kinase) (for example, Y-27632 or Fasudil (HA-1077)) is also added is preferably used. The concentration of Y-27632 used as the ROCK inhibitor is, for example, about 1 µM to about 50 µM. The optimum concentration can be set through preliminary experiments.

The ROCK inhibitor strongly inhibits cell death when cells are in a dispersed state. Therefore, instead of using the ROCK inhibitor over the entire culture period of step (2), the cells may be treated in a medium containing the ROCK inhibitor only when seeding cells (i.e. at the start of culture) or when collecting and dispersing cells, for example, for subculture.

Preferably, a medium having a ciliary neurotrophic factor (CNTF), a brain-derived neurotrophic factor (BDNF), a neurotrophin 3 (NT-3), a fetal bovine serum, an N2 supplement, a B27 supplement, or the like added is used so that the use thereof is advantageous in differentiation induction along the neuron lineage. Incidentally, the N2 supplement is available from Gibco (product name N2 supplement (x100)) or the like, and the B27 supplement is available from Gibco (product name B27 supplement (x100)) or the like.

Furthermore, any other component may be added to the medium as needed. Examples of the component which can be added include insulin, an iron source (e.g., transferrin), a mineral (e.g., sodium selenate), a saccharide (e.g., glucose), an organic acid (e.g., pyruvic acid or lactic acid), a serum protein (e.g., albumin), an amino acid (e.g., L-glutamine), a reducing agent (e.g., 2-mercaptoethanol), a vitamin (e.g., ascorbic acid or d-biotin), an antibiotic (e.g., streptomycin, penicillin, or gentamicin), and a buffer (e.g., HEPES).

In this step (2), suspension culture is performed to form a neurosphere. For example, Serum-free Floating culture of Embryoid Body-like aggregates with quick reaggregation (SFEB method/SFEBq method; Watanabe et al., Nature Neuroscience 8,288-296 (2005), WO 2005/123902), neurosphere method (Reynolds BA and Weiss S., Science, USA, 1992 Mar 27; 255 (5052): 1707-10) and the like can be employed.

In the present invention, step (2) is performed under normal oxygen partial pressure. In cell culture, the condition of a lowered oxygen concentration (low oxygen partial pressure/low oxygen concentration) may sometimes be used in consideration of the environment in the living body. However, the condition "under normal oxygen partial pressure" in the present invention is in contrast to such a special condition. That is, the condition "under normal oxygen partial pressure" is a condition in which the oxygen concentration is not intentionally adjusted. The oxygen concentration in the case of the condition "under normal oxygen partial pressure" is typically about 18% to about 22%, though it may vary depending on other conditions (humidity, coexisting CO₂ concentration, and the like).

Performing step (2) under normal oxygen partial pressure eliminates the need to set a special oxygen condition (typically, low oxygen environment) throughout the entire culture period (steps (1) to (3)), and can suppress induction of differentiation into unnecessary cells such as glial cells, and thus is a highly practical preparation method.

Other culture conditions (culture temperature, CO₂ concentration, and the like) can be set as appropriate. The culture temperature is, for example, about 30 to 40°C, preferably about 37°C. The CO₂ concentration is, for example, about 1 to 10%, preferably about 5%.

The period of step (2) (culture period) is, for example, 7 days to 21 days, preferably 10 days to 16 days. If the culture period is too short or too long, the differentiation efficiency may be lowered.

The formed neurosphere may be collected to dissociate, and then the dissociated cells may be subjected to further suspension culture. That is, subculture may be performed. However, the number of subcultures is preferably small, and the number of subcultures is set to 1 or 0 (that is, no subculture is performed). Such a small number of subcultures is advantageous in the preparation of dopamine neurons in a short period of time, and is considered to be also effective in avoiding promotion of unintended differentiation induction (for example, induction of differentiation into glial cells). On the other hand, since subculture is effective in improving the cell purity, it can be said that the subculture is optimally performed once. When subculture is performed once, the subculture is preferably performed on the sixth to tenth days from the start of step (2). In addition, when collecting the neurosphere during subculture, it is preferable to prevent contamination of the cells adhering to the surface of the culture vessel. Such an operation can contribute to the improvement of the preparation efficiency and purity of dopamine neurons.

### Step (3)

The neurosphere formed by step (2) contains undifferentiated cells of the nervous system and undifferentiated cells of the midbrain system. In step (3), the cells constituting the neurosphere are collected and induced differentiation into dopamine neurons. Alternatively, the neurosphere is directly (i.e. as a cell aggregate) induced into dopamine neurons. Media and culture conditions suitable for inducing differentiation into dopamine neurons are known. Regarding basic culture methods and operations, a protocol provided by ThermoFisher (published on the ThermoFisher website) or the like can be referred to. Specifically, for example, differentiation into dopamine neurons is induced by adherent culture in a medium containing a γ-secretase inhibitor, a neurotrophic factor, ascorbic acid, TGF-β3 and cAMP or a cAMP analog. Preferably, the γ-secretase inhibitor used is N-[N-(3,5-difluorophenacetyl-L-alanyl)]-S-phenylglycine t-butyl ester, the neurotrophic factor used is a brain-derived neurotrophic factor (BDNF) and a glial cell-derived neurotrophic factor (GDNF), and the cAMP analog used is diptyryl cAMP.

In one embodiment, the neurosphere formed in step (2) is collected to dissociate (to form single cells), and the dissociated cells are seeded in a culture vessel and cultured. In another embodiment, instead of such dispersion culture, the collected neurosphere is subjected to adherent culture as a cell aggregate. In this case, normally, if culture is continued, some of cells migrate from the neurosphere to the surrounding, and dopamine neurons can be observed in the migrated cells.

For example, dishes, Petri dishes, tissue culture dishes, multi-dishes, microplates, microwell plates, multi-plates, multi-well plates, chamber slides, laboratory dishes, and the like can be used in this adherent culture. In order to enhance the adhesiveness of the cells to the culture surface, it is preferable to use a culture vessel coated with Matrigel^{™} (BD), poly-D-lysine, poly-L-lysine, collagen, gelatin, laminin, heparan sulfate proteoglycan, entactin, or a combination of two or more thereof.

Other culture conditions such as culture temperature, CO₂ concentration, and O₂ concentration can be set as appropriate. The culture temperature is, for example, about 30 to 40°C, preferably about 37°C. The CO₂ concentration is, for example, about 1 to 10%, preferably about 5%. Moreover, culture may be performed under normal oxygen partial pressure.

The period (culture period) of step (3) is not particularly limited. For example, the culture is performed for 5 days or longer, preferably 7 days or longer. Although culture for an excessively long period can cause exhaustion of cells, decrease in activity, cell death, or the like, differentiation/maturation generally progresses as the culture period is longer. Therefore, the culture period is, for example, set to 5 days to 21 days, although the upper limit of the culture period in this step is not particularly limited. The cells may be subjected to subculture as necessary. For example, the cells are collected at a stage where they are brought in a subconfluent or confluent state, a part of the cells is seeded in another culture vessel, and culture is continued.

By step (3), dopamine neurons are obtained. Dopamine neurons can be identified or confirmed using, as an index, expression of dopamine markers (tyrosine hydroxylase, dopamine transporter), FOXA2 as a midbrain marker, or the like, or by evaluation of the dopamine production ability.

Dopamine neurons can be obtained from pluripotent stem cells in about 21 days to 30 days, though it may vary depending on the type and state of the cells used and the culture conditions in each step.

In step (i), the prepared cell population is cultured in the presence of the test substance, and the culture conditions may be general conditions suitable for survival and proliferation of the cells to be used. However, special culture conditions may be adopted, for example, in the presence of a substance that inhibits cell movement ability (for example, a Rho signal stimulant). Besides two-dimensional culture, three-dimensional culture using a scaffold material such as a hydrogel (an animal extracellular matrix extraction hydrogel, a protein hydrogel, a peptide hydrogel, or a polymer hydrogel) can also be adopted. Those skilled in the art can refer to the teachings in the present specification (especially, description of the Examples) and known information (for example, research reports and reviews using the cells to be used or cells equivalent/similar thereto) to set appropriate culture conditions. The optimum culture conditions can be determined through preliminary experiments.

The test substance in step (i) is not particularly limited. Various substances whose efficacy or toxicity is required to be evaluated can be test substances. As the test substance, organic or inorganic compounds having various molecular sizes can be used. Examples of the organic compounds include nucleic acids, peptides, proteins, lipids (simple lipids, complex lipids (phosphoglycerides, sphingolipids, glycosyl glycerides, cerebrosides, etc.), prostaglandins, isoprenoids, terpenes, steroids, polyphenols, catechins, and vitamins (B 1, B2, B3, B5, B6, B7, B9, B12, C, A, D, E, etc.). Existing or candidate components such as pharmaceuticals, nutritional foods, food additives, agricultural chemicals, and cosmetics are also preferred test substances. A plant extract, a cell extract, a culture supernatant, or the like may be used as the test substance. By simultaneously adding two or more kinds of test substances, interactions, synergistic actions, and the like between the test substances may be examined. The test substance may be derived from natural products or obtained by synthesis. In the latter case, for example, an efficient assay system can be constructed using a combinatorial synthesis technique.

After the start of culture, the positions of the individual cells that have moved are measured. In the evaluation method of the present invention, cells that have moved among the cells constituting the cell population, i.e., cells that have moved from the positions at the start of culture to the surroundings are to be measured. It is another feature of the present invention that the position of each of the cells is measured. It is not essential that all of the cells that have moved should be measured. However, in order to determine a high-quality movement pattern that provides more useful evaluation results, preferably 50% or more, more preferably 60% or more, still more preferably 70% or more, even more preferably 80% or more of the cells that have moved are to be measured. In measuring the positions of the cells, an optical microscope, a fluorescence microscope, a still camera, a video camera, and the like can be combined as appropriate to enable measurement over time.

If the cells to be measured (that is, cells that have movement ability and constitute the cell population) are labeled in advance, the positions of the cells can be measured using the label as an index. Examples of the label herein can include fluorescent labels such as GFP and RFP.

The position of each of the cells is measured multiple times over time (usually, continuously along the time axis) so that a more suitable movement pattern can be determined for the evaluation of efficacy or toxicity. That is, the cell is tracked and its position is continuously measured and recorded. Thus, it becomes possible to grasp a change over time in cell movement direction. The interval at continuous measurement is, for example, several seconds to several hours, preferably several minutes to several tens of minutes (for example, 1 minute, 2 minutes, 3 minutes, 4 minutes, 5 minutes, 10 minutes, 15 minutes, 20 minutes, or 25 minutes). The measurement interval defines the quantity and quality of the obtained position information, and thus affects the quality of the movement pattern determined based on the position information. In order to create a higher quality movement pattern, it is desirable to shorten the measurement interval as much as possible. However, if an excessively short measurement interval is adopted, the amount of data and the data processing time will become enormous, which can cause practical problems. Therefore, it is preferable to set an appropriate measurement interval in consideration of the number of the cells to be measured and the processing capability of the measuring instruments and data analysis software to be used, on the condition that a movement pattern of sufficiently high quality based on which efficacy or toxicity can be evaluated (step (iii)) can be obtained. Software such as Particle Tracker available from Fiji can be used for measurement or tracking over time.

When continuous measurement is performed, the timing of starting the measurement is not particularly limited, and the measurement may be started either before or after the cells start to move. Preferably, the measurement is started before or immediately after the cells start to move, so that the movement process can be recorded from the beginning. The timing of starting the measurement may be set depending on the elapsed time after the start of culture. For example, the measurement is started at the time when several hours have elapsed after the start of the culture.

In the case of continuous measurement, the time from the initial measurement to the final measurement (referred to also as "tracking time") is also not particularly limited. For example, the tracking time can be set within the range of 4 hours to 12 hours.

Step (ii) involves analyzing the variations in movement of the respective cells from information on the measured positions, totalizing the analysis results, and determining a movement pattern of the cell population. For example, a distance ratio calculated in the following (A) is used for the analysis of the movement variations (see Fig. 1).
(A) The distance ratio d(t)/D(t) is calculated, where D(t) is a total movement distance of a cell at a measurement time t and d(t) is a linear distance between two points, i.e., a movement start point and a movement end point.

The distance ratio calculated in (A) reflects the variation in movement direction. A smaller distance ratio value means a greater variation (fluctuation) in movement direction. The movement pattern is determined by totalizing the analysis results (in this case, distance ratio) for the individual cells. At the time of totalization, statistical processing (for example, an average value and a standard deviation or standard error is used) is usually performed. As a result of totalization, if the value of the distance ratio tends to be small, the movement pattern has a great variation (fluctuation) in movement direction, and if the value of the distance ratio tends to be large, the movement pattern has a small variation (fluctuation) in movement direction.

By measuring the cell position over time in step (i) and calculating the distance ratio of (A) at each measurement time, it is possible to grasp a change over time in distance ratio (that is, variation in movement direction).

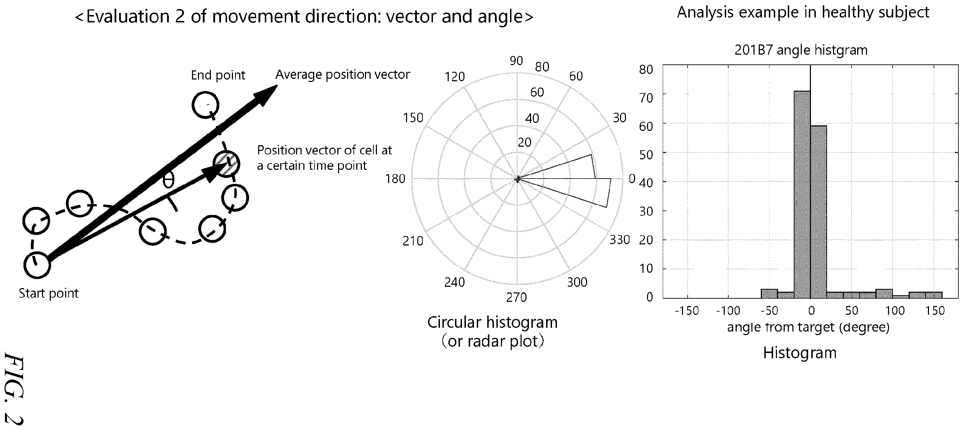

In another aspect, an angle θ obtained in the following (B) is used for the analysis of the movement variations (see Fig. 2).

(B) A measurement time interval t/n (where n is an integer of 2 or more) is set with respect to the measurement time t to measure the position of the cell, thereby determining the angle θ between a position vector of the cell and a reference axis at each time point.

In (B), a position vector that reflects the positional relationship of a cell at a certain time point is determined using the position of a reference cell at the start of measurement ((t/n) × 0) as a reference point, and the angle θ with the reference axis is calculated and used. First, the position of the cell at the start of measurement is set as the reference point (at the start). The measurement time interval t/n (where n is an integer of 2 or more) is set with respect to the measurement time t, and the position vector of the cell is measured at each measurement ((t/n) × 1, (t/n) × 2, (t/n) × 3, ... (t/n) × n). Next, the angle θ between the position vector of the cell at each measurement and the reference axis (typically, a straight line passing through the reference point is used as the reference axis) is determined. The values of the angle θ (n values can be obtained per cell) for the individual cells thus obtained are totalized for all the cells to be measured to create the frequency distribution of the angle θ. The reference axis can be arbitrarily set. For example, the above-obtained position vectors of the cell at the respective measurements (that is, position vectors of the cell at (t/n) × 1, (t/n) × 2, (t/n) × 3, ... (t/n) × n) are summed, and the sum is divided by n to calculate an average position vector, which is used as the reference axis. The reference point and the reference axis are set for each cell.

The measurement time interval is, for example, 10 seconds to 120 minutes. Further, the integer n that defines the measurement time interval has only to be determined in relation with the total measurement time (i.e., predetermined time t). For example, t is 1 hour to 240 hours, and 2 ≤ n ≤ 1000000.

In the case of (B), the frequency distribution of the angle θ defines the movement pattern. The variations in angle θ and movement direction of the cell show a positive correlation. Therefore, when the frequency is high in a range of small angles (θ), the movement pattern has a small variation (fluctuation), and when the frequency is high in a range of large angles (θ), the movement pattern has a great variation (fluctuation). A histogram may be created to determine the movement pattern based on the shape thereof. Alternatively, the variability of the angle θ from the average value may be examined, and a median or average value of the variability may be used to determine the degree or tendency of the variation (fluctuation).

In still another aspect, a velocity calculated in the following (C) is used for the analysis of the movement variations.

(C) The velocity D(t)/t is calculated, where D(t) is the total movement distance of a cell at a measurement time t.

The velocity also reflects variations in movement of the cell. The deviation of the velocity at a certain time or the velocity change over time from the normal pattern indicates an abnormality in movement variation.

The movement pattern can be expressed in the form of a table, a graph, a plot, or the like. For example, if a plurality of sections is provided for the range of distance ratio values and the degree of variation is associated with each of the sections using a number or symbol, the level of the variation in movement direction can also be expressed by the number or symbol.

In step (iii) subsequent to step (ii), the efficacy or toxicity of the test substance is determined based on the determined movement pattern. As described above, the method of the present invention can be used as an efficacy evaluation system or toxicity evaluation system. In the former case, the efficacy of the test substance is determined in this step, and in the latter case, the toxicity of the test substance is determined in this step. The characteristics of each of the evaluation systems will be described below.

### <Efficacy evaluation system>

In one aspect of the present invention, the evaluation method of the present invention is configured as a means for evaluating the efficacy of the test substance, that is, a drug evaluation system. According to this aspect, it becomes possible to screen for a pharmaceutical component or candidate therefor that is effective against various diseases of which the onset and progress are caused at least by cell migration disorder (target diseases).

In this aspect, typically, abnormal cells are used as the cells in step (i). Then, in step (iii), the efficacy of the test substance is determined using normalization of the movement pattern determined in step (ii) as an index. The "normalization of the movement pattern" means improvement in abnormal movement pattern exhibited by a population of abnormal cells. When the movement pattern is normalized, it is closer to the movement pattern of the corresponding normal cells (usually, movement pattern with a small variation (fluctuation)). Therefore, the effectiveness of the test substance is supported by the fact that the movement pattern determined in step (ii) (referred to as "movement pattern of the test group") is closer to the movement pattern of the corresponding normal cells (usually, movement pattern with a small variation (fluctuation)), as compared with the movement pattern determined according to the same procedures except that the cells are cultured in the absence of the test substance (referred to as "movement pattern of the control group"), i.e., the movement pattern determined in step (ii) has approximated the movement pattern of the corresponding normal cells due to the presence (influence) of the test substance. By comparing or contrasting the three movement patterns, i.e., the movement pattern of the test group, the movement pattern of the control group, and the movement pattern of the normal group, it can be grasped whether or not the movement pattern has been normalized, and the effectiveness of the test substance can be determined based on the result. Moreover, the degree of normalization of the movement pattern reflects the degree of effectiveness (efficacy), and thus the degree or potency of effectiveness can also be determined based on the degree of normalization. It can be said that a test substance which has been confirmed to be effective is promising as a pharmaceutical component or candidate therefor for the disease caused by cell movement disorder.

The "corresponding normal cell" is a cell of the same type, which is typically derived from a healthy subject. For example, when the abnormal cells are dopamine neurons derived from a patient suffering from schizophrenia, the corresponding normal cells are dopamine neurons derived from a healthy subject (typically, dopamine neurons obtained by inducing differentiation of iPS cells produced from peripheral blood collected from a healthy subject). As another example, when the abnormal cells are glutamatergic neurons derived from a patient suffering from Rett syndrome, the corresponding normal cells are glutamatergic neurons derived from a healthy female.

### <Toxicity evaluation system>

In another aspect of the present invention, the evaluation method of the present invention is configured as a means for evaluating the toxicity of the test substance, that is, a toxicity evaluation system. According to this aspect, whether or not a test substance (for example, a pharmaceutical component or candidate therefor, a dietary supplement (supplement) or candidate therefor, or a food additive or candidate therefor) affects the cell movement ability and is thus toxic, or the degree of toxicity can be evaluated.

In this aspect, normal cells or abnormal cells are used as the cells in step (i). In step (iii) when normal cells are used, the toxicity of the test substance is determined using abnormalization of the movement pattern determined in step (ii) as an index. The abnormalization of the movement pattern means that the movement pattern changes, that is, changes into a movement pattern different from the normal movement pattern. Therefore, typically, the toxicity of the test substance is supported by the fact that, when the movement pattern determined in step (ii) (movement pattern of the test group) is compared with the movement pattern determined according to the same procedures except that the cells are cultured in the absence of the test substance (movement pattern of the control group), they are different from each other. Further, the degree of change in movement pattern reflects the degree of toxicity, and thus the degree or potency of toxicity can also be determined based on the degree of difference.

Appropriate cells are preferably selected as normal cells in consideration of the purpose of toxicity evaluation. For example, in the case of evaluating the neurotoxicity of the test substance (however, the neurotoxicity resulting from the influence on the cell movement ability is to be evaluated), neurons (dopamine neurons, glutamatergic neurons, serotonin neurons, GABAergic neurons, etc.) are selected.

On the other hand, in step (iii) when abnormal cells are used, the toxicity of the test substance is determined using further abnormalization of the movement pattern determined in step (ii) as an index. The "further abnormalization" means that the difference from the normal movement pattern becomes more remarkable, and when further abnormalization occurs, the movement pattern typically has a greater variation (fluctuation) in movement direction. By comparing or contrasting the three movement patterns, i.e., the movement pattern determined in step (ii) (movement pattern of the test group), the movement pattern determined according to the same procedures except that the cells are cultured in the absence of the test substance (movement pattern of the control group), and the movement pattern of the corresponding normal cells (movement pattern of the normal group), whether or not further abnormalization of the movement pattern has occurred, or the degree of abnormalization can be determined.

For example, cells derived from a patient may be used as the abnormal cells. If diseased cells derived from a patient suffering from a specific disease are adopted as the abnormal cells, the evaluation system can be used to evaluate the toxicity of a therapeutic drug targeting the disease or a candidate therefor. Such an evaluation system is useful, for example, as a means for evaluating a side effect of a therapeutic drug or candidate therefor.

As is clear from the above description, the "movement pattern defined by variations in movement direction of individual cells constituting a cell population" used as an index in the drug evaluation method of the present invention reflects the state of the cells. Focusing on this point, the cell movement pattern is also useful for evaluating the quality (normality, abnormality, homogeneity, etc.) of the cell population. Therefore, the present invention also provides, as another aspect, a method for evaluating the quality of a cell population based on a cell-based assay system to obtain a movement pattern defined by directional movement ability of individual cells constituting a cell population as characterized in the claims.

### [Examples]

The following studies were carried out with the aim of developing a novel evaluation system useful in researches on the onset mechanism and pathologic conditions of a central nervous system disease, development of a therapeutic method, and the like.

### 1. Material and method

### (1) Persons from which iPS cells were established

1 RELN-deleted person (RELN-del1)
1 healthy male (CON2)

### (2) Establishment of iPS cell

A healthy female control (CON1) was obtained from RIKEN BioResource Research Center (BRC). The absence of genomic abnormalities including RELN deletion was confirmed in advance. iPS cells from the RELN-deleted person were established, using episomal vectors, from peripheral lymphocytes in accordance with the previous report (Okita, K. et al. A more efficient method to generate integration-free human iPS cells. Nature methods 8, 409-412 (2011)). The established iPS cells were cultured on feeder cells (mitomycin-C-treated mouse embryonic fibroblasts: MEFs) using an iPS cell medium (DMEM/F12 containing 20% KSR, 2 mM L-glutamine, 0.1 mM non-essential amino acids, 2-mercaptoethanol, penicillin/streptomycin, and bFGF). In feeder-free culture, the iPS cells were cultured on a culture dish coated with Matrigel^{™} (BD). As the medium, an iPS cell medium (MEF-conditioned medium) exposed to feeder cells overnight was used.

### (3) Preparation of RELN-deleted isogenic iPS cell strain

Using the CRISPR/Cas9 system, RELN-deleted isogenic iPS cells (Isogenic(- /-) and Isogenic(+/-)) were artificially produced from the healthy control (CON1). A Cas9 expression vector and an sgRNA expression vector were obtained from Addgene. The Cas9 expression vector and sgRNA expression vector were co-transfected using Lipofectamine 3000, and selection with puromycin was performed after 48 hours. After pretreatment with 10 µM Y-27632, the iPS cells were dispersed using TrypLE ^{™} select (Thermo Fisher Scientific Inc.). Thereafter, the Cas9 expression vector and sgRNA expression vector were co-transfected using FuGENE (registered trademark) HD (Promega), and the cells were seeded on a 6-well plate coated with Matrigel^{™} (BD) at 1 × 10⁶ cells/well. Selection with puromycin was performed after 24 hours.

### (4) Formation of neurosphere

Neurospheres were formed from the healthy subject iPS cells, RELN-deleted patient iPS cells, and RELN-deleted isogenic iPS cells in accordance with the following protocol. First, iPS cells were cultured in an iPS cell medium to which SB431542 (3 µM), CHIR99021 (3 µM), and dorsomorphin (3 µM) were added for 7 days (from Day 0 to Day 7). Then, the iPS cells which were dispersed by TrypLE^{™} select (Thermo Fisher Scientific Inc.) and caused to pass through a cell strainer were cultured in suspension in a neurosphere medium (a medium (MHM medium) obtained by adding, to DMEM/F12, 1 × N2 supplement, 0.6% glucose, penicillin/streptomycin, and 5 mM HEPES, to which 1 × B27 supplement, 20 ng/ml bFGF, 10 ng/ml human LIF, 10µM Y27632, 3µM CHIR99021, 2µM SB431542, 100 ng/ml FGF8, and 1µM purmorphamine were added) for 2 weeks to form neurospheres (Day 7 to Day 21). FGF8 and purmorphamine were added from Day 10. On Day 14, the neurospheres were collected and dispersed to form single cells, and then the cells were cultured in suspension again to form neurospheres again (secondary neurospheres).

### (5) Movement ability test of neuron

Secondary neurospheres (Day 21) were seeded one by one onto a Matrigel ^{™} (BD) coated culture dish, and cultured in a medium for dopamine neurons (obtained by adding, to an MHM medium, B27 supplement, 10 µM DAPT, 20 ng/ml BDNF, 20 ng/GDNF, 0.2 mM ascorbic acid, 1 ng/ml TGF-β3, and 0.5 mM dbcAMP). A video was shot with IncuCyte (registered trademark) (ESSEN BIOSCIENCE). For cell tracking, images were shot continuously every 15 minutes for a total of 4 hours, 48 hours to 52 hours after seeding, and analyzed using ImageJ (time-lapse analysis). The movement distance was calculated based on the XY coordinates at each shooting point. Differentiation into dopamine neurons was performed by a method obtained by improving the previously reported method (Fujimori, K. et al. Modeling neurological diseases with induced pluripotent cells reprogrammed from immortalized lymphoblastoid cell lines. Molecular brain 9, 88 (2016)).

### (6) Evaluation 1 of movement method (distance ratio) (see Fig. 1)

The movement direction was evaluated by the ratio between the total movement distance of a cell and the linear distance between two points, i.e., a start point and an end point.

### (7) Evaluation 2 of movement method (vector and angle) (see Fig. 2)

The movement direction was evaluated by the angle between the position vector of a cell and the reference axis (average position vector). The position vector measurement interval was set to 15 minutes.

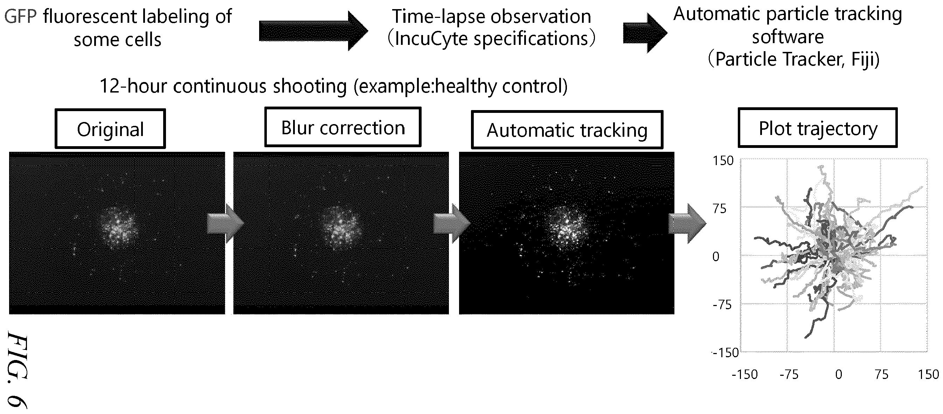

### (8) Automatic detection method (see Fig. 6)

Some of the cells constituting neurospheres were labeled with GFP. Specifically, CellLight Reagent (Thermo Fisher) was used. The labeled neurospheres were subjected to a movement ability test, time-lapse observed (using IncuCyte), and analyzed by automatic particle software (Particle Tracker, Fiji). According to this method, there is no bias due to an artificial operation for automatic detection. Also, the XY coordinate axis values can be used to quantify the neuronal dynamics.

### (9) Statistical analysis

For comparison of the average values, the Student t-test (two-sided test) was used for comparison between two groups, and the Dunnett's method was used after the ANOVA test for comparison among three groups. Comparison of the distributions was made using the Pearson's Chi-square test. In all cases, p < 0.05 was considered significant.

### 2. Results

### (1) Movement pattern of healthy subject

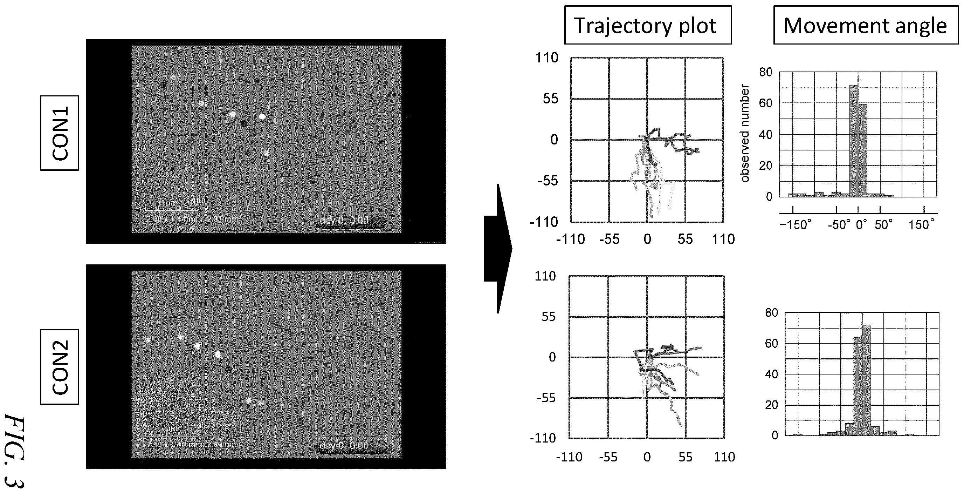

The positions of dopamine neurons induced from the healthy subject iPS cells were tracked, and the movement pattern focusing especially on the directional property was evaluated according to the above "Evaluation 2 of movement method (vector and angle)" (Fig. 3). The healthy subject-derived neurons showed a constant movement pattern (characterized by straightness) even *in vitro.* There was almost no difference in rule of the directional property among the individual cells.

### (2) Comparison between movement pattern of RELN deletion and movement pattern of healthy subject

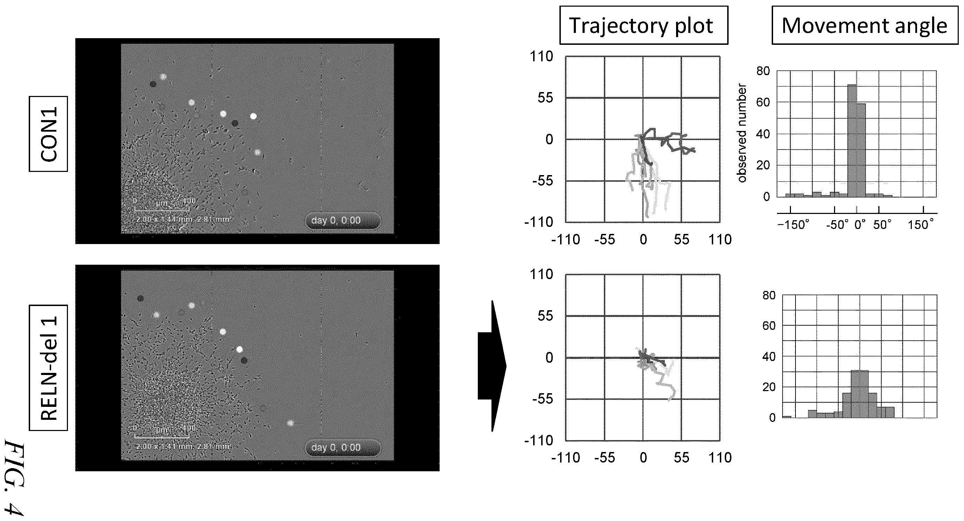

The positions of dopamine neurons induced from the RELN-deleted patient iPS cells were tracked, and the movement pattern was evaluated according to the above "Evaluation 2 of movement method (vector and angle)" (Fig. 4). The RELN-deleted patient-derived neurons showed a pattern in which the individual cells were different in movement direction, in contrast to the healthy subject-derived neurons. That is, an abnormality in the movement pattern could be detected.

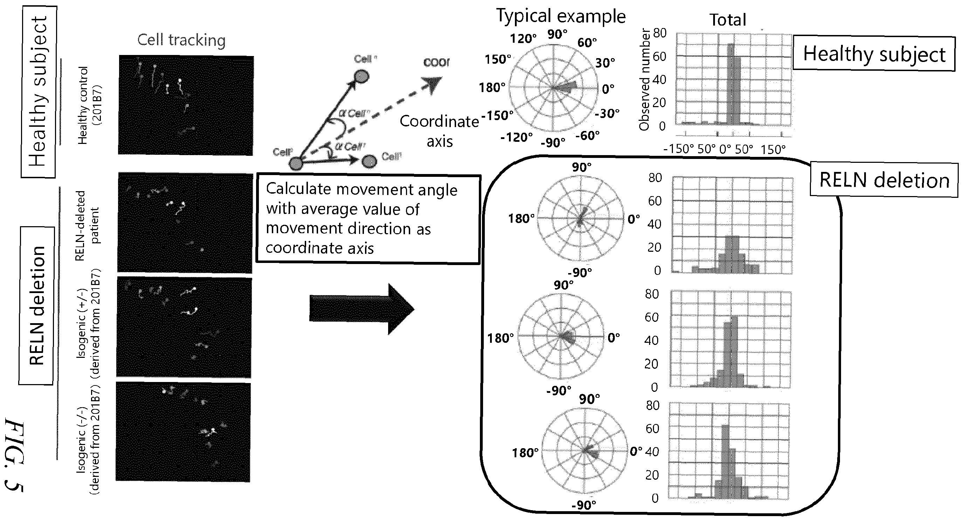

The movement patterns of the dopamine neurons induced from RELN-deleted isogenic iPS cells were also analyzed, and the movement pattern of the healthy subjects and the movement patterns of the RELN deletion (RELN-deleted patient and RELN-deleted isogenic (Isogenic(-/-) and Isogenic (+/-)) were compared with each other (Fig. 5). The healthy subject shows a movement pattern with a small variation (fluctuation) in which the angle θ is distributed in a narrow range (about 80% of the angle θ falling within the range of-20° to +20°). In contrast, the movement patterns of the RELN deletion have a great variation, with only about 45% to 65% of the angle θ falling within the range of -20° to +20°.

### (3) Evaluation by automatic detection system

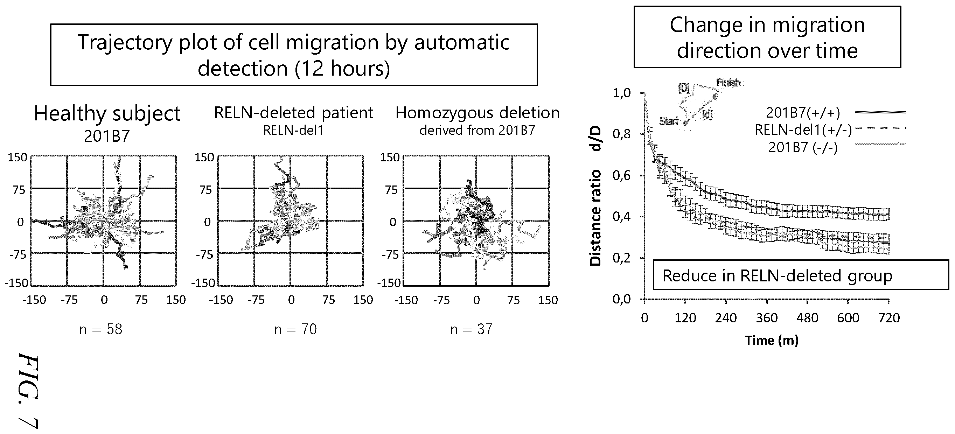

In consideration of applications to drug evaluation systems, a measurement system (automatic detection system) for automatically detecting/quantifying movement patterns was constructed (Fig. 6) and the effectiveness thereof was verified. The healthy subject-derived neurons, RELN-deleted patient-derived neurons, and RELN-deleted isogenic-derived neurons were subjected to trajectory plotting (12 hours) by automatic detection, and the movement patterns were compared and evaluated according to the above "Evaluation 1 of movement method (distance ratio)" (Fig. 7). A clear decrease in distance ratio was observed in the RELN-deleted groups (RELN-del1(+/-) and 201B7(-/-)) as compared with the healthy subject (201B7(+/+)).

As described above, it was possible to detect an abnormality in movement pattern characteristic of the RELN-deleted groups also by the automatic detection system (reproducibility was obtained). In addition, it became possible to measure the movement phenomenon of the individual cells over a long period of time.

### [Industrial Applicability]

Since the movement ability of cells is important for normal formation and regeneration processes of various tissues and organs, the drug evaluation system of the present invention using the cell movement pattern as an index can be widely used and applied. For example, the present invention can be expected to be utilized for: pathological researches on central nervous system diseases, basic researches on neurogenesis and circuit formation, development of central nervous system drugs, development of therapeutic methods/therapeutic drugs for spinal cord injury, peripheral neuropathy, and organ formation (for example, congenital heart disease), and prediction of the effects of drugs taken by a pregnant or nursing mother on the fetus/child brain and organ formation.

## Claims

1. A drug evaluation method using a cell-based assay system to obtain a movement pattern defined by directional movement ability of individual cells constituting a cell population as an index, wherein the cells are neurons derived from induced pluripotent stem cells.

2. The method according to claim 1, comprising the following steps (i) to (iii) of:
(i) preparing a population of cells exhibiting movement ability, starting culture in the presence of a test substance, and then measuring positions of the individual cells that have moved over time,
(ii) analyzing the directional movement ability of the respective cells from information on the measured positions, totalizing the analysis results, and determining a movement pattern of the cell population, and
(iii) determining efficacy or toxicity of the test substance based on the determined movement pattern.

3. The method according to claim 2, wherein a distance ratio calculated in the following (A), an angle θ determined in the following (B), or a velocity calculated in the following (C) is used for the analysis of step (ii):
(A) calculating the distance ratio d(t)/D(t), where D(t) is a total movement distance of each of the cells at a measurement time t and d(t) is a linear distance between two points, i.e., a movement start point and a movement end point,
(B) setting a measurement time interval t/n, where n is an integer of 2 or more, with respect to the measurement time t to measure a position of each of the cells, thereby determining the angle θ between a position vector of each of the cells and a reference axis at each time point, and
(C) calculating the velocity D(t)/t, where D(t) is the total movement distance of each of the cells at the measurement time t.

4. The method according to claim 3, wherein an average position vector calculated from a movement trajectory of each of the cells up to the time t is used as the reference axis of (B).

5. The method according to any one of claims 2 to 4, wherein the cells of step (i) are abnormal cells, and, in step (iii), the efficacy of the test substance is determined using normalization of the movement pattern as an index.

6. The method according to any one of claims 2 to 4, wherein the cells of step (i) are normal cells, and, in step (iii), the toxicity of the test substance is determined using abnormalization of the movement pattern as an index.

7. The method according to any one of claims 2 to 4, wherein the cells of step (i) are abnormal cells, and, in step (iii), the toxicity of the test substance is determined using further abnormalization of the movement pattern as an index.

8. The method according to claim 5 or 7, wherein the abnormal cells are diseased cells having genetic characteristics of a target disease.

9. The method according to claim 8, wherein the diseased cells are patient-derived cells or cells prepared by genetic engineering.

10. The method according to claim 9, wherein the patient-derived cells are cells obtained by inducing differentiation of induced pluripotent stem cells prepared from patient cells.

11. The method according to any one of claims 1 to 10, wherein the neurons are dopamine neurons, glutamatergic neurons, serotonin neurons, or GABAergic neurons.

12. The method according to claim 11, wherein the dopamine neurons are prepared by the method comprising the following steps (1) to (3):
(1) culturing pluripotent stem cells in the presence of a TGF-β family inhibitor, a GSK3β inhibitor, and a BMP inhibitor;
(2) suspension-culturing the cells obtained in step (1) in the presence of a TGF-β family inhibitor, a GSK3β inhibitor, FGF8, and a hedgehog signal agonist and under normal oxygen partial pressure to form a neurosphere; and
(3) collecting the cells constituting the neurosphere to induce differentiation thereof into dopamine neurons, or directly inducing the neurosphere into dopamine neurons.

13. A method for evaluating a quality of a cell population using a cell-based assay system to obtain a movement pattern defined by directional movement ability of individual cells constituting a cell population as an index, wherein the cells are neurons derived from induced pluripotent stem cells.

14. Use of an automatic detection system for automatically detecting and/or quantifying movement patterns in the method of any one of claims 1 to 13.

## Patentansprüche

1. Arzneimittelbewertungsverfahren, das ein zellbasiertes Assaysystem verwendet, um ein Bewegungsmuster zu erhalten, das durch die Richtungsbewegungsfähigkeit einzelner Zellen, die eine Zellpopulation bilden, als Index definiert ist, wobei die Zellen Neuronen sind, die aus induzierten pluripotenten Stammzellen stammen.

2. Verfahren nach Anspruch 1, umfassend die folgenden Schritte (i) bis (iii):
(i) Herstellung einer Population von Zellen, die die Fähigkeit zur Bewegung aufweisen, Beginn der Kultur in Gegenwart einer Testsubstanz und anschließende Messung der Positionen der einzelnen Zellen, die sich im Laufe der Zeit bewegt haben,
(ii) Analysieren der Richtungsbewegungsfähigkeit der jeweiligen Zellen aus Informationen über die gemessenen Positionen, Summieren der Analyseergebnisse und Bestimmen eines Bewegungsmusters der Zellpopulation, und
(iii) Bestimmung der Wirksamkeit oder Toxizität der Testsubstanz auf der Grundlage des ermittelten Bewegungsmusters.

3. Verfahren nach Anspruch 2, wobei ein im Folgenden (A) berechnetes Abstandsverhältnis, ein im Folgenden (B) ermittelter Winkel θ oder eine im Folgenden (C) berechnete Geschwindigkeit für die Analyse von Schritt (ii) verwendet wird:
(A) Berechnung des Abstandsverhältnisses d(t)/D(t), wobei D(t) der Gesamtbewegungsabstand jeder Zelle zu einem Messzeitpunkt t und d(t) ein linearer Abstand zwischen zwei Punkten, d.h. einem Bewegungsstartpunkt und einem Bewegungsendpunkt, ist,
(B) Einstellen eines Messzeitintervalls t/n, wobei n eine ganze Zahl von 2 oder mehr ist, in Bezug auf die Messzeit t, um eine Position jeder der Zellen zu messen, wodurch der Winkel θ zwischen einem Positionsvektor jeder der Zellen und einer Referenzachse zu jedem Zeitpunkt bestimmt wird, und
(C) Berechnung der Geschwindigkeit D(t)/t, wobei D(t) die Gesamtbewegungsstrecke jeder der Zellen zum Messzeitpunkt t ist.

4. Verfahren nach Anspruch 3, wobei ein durchschnittlicher Positionsvektor, der aus einer Bewegungstrajektorie jeder der Zellen bis zum Zeitpunkt t berechnet wird, als Referenzachse von (B) verwendet wird.

5. Verfahren nach einem der Ansprüche 2 bis 4, wobei es sich bei den Zellen aus Schritt (i) um abnorme Zellen handelt und in Schritt (iii) die Wirksamkeit der Testsubstanz unter Verwendung der Normalisierung des Bewegungsmusters als Index bestimmt wird.

6. Verfahren nach einem der Ansprüche 2 bis 4, wobei es sich bei den Zellen aus Schritt (i) um normale Zellen handelt und in Schritt (iii) die Toxizität der Testsubstanz unter Verwendung der Abnormalisierung des Bewegungsmusters als Index bestimmt wird.

7. Verfahren nach einem der Ansprüche 2 bis 4, wobei es sich bei den Zellen aus Schritt (i) um abnorme Zellen handelt und in Schritt (iii) die Toxizität der Testsubstanz anhand einer weiteren Abnormalisierung des Bewegungsmusters als Index bestimmt wird.

8. Verfahren nach Anspruch 5 oder 7, wobei die abnormen Zellen kranke Zellen mit genetischen Merkmalen einer Zielkrankheit sind.

9. Verfahren nach Anspruch 8, wobei die erkrankten Zellen vom Patienten stammende oder gentechnisch hergestellte Zellen sind.

10. Verfahren nach Anspruch 9, wobei die vom Patienten stammenden Zellen Zellen sind, die durch Induktion der Differenzierung von induzierten pluripotenten Stammzellen, die aus Patientenzellen hergestellt wurden, erhalten wurden.

11. Verfahren nach einem der Ansprüche 1 bis 10, wobei die Neuronen Dopamin-Neuronen, glutamaterge Neuronen, Serotonin-Neuronen oder GABAerge Neuronen sind.

12. Verfahren nach Anspruch 11, wobei die Dopamin-Neuronen durch das Verfahren hergestellt werden, das die folgenden Schritte (1) bis (3) umfasst:
(1) Kultivierung pluripotenter Stammzellen in Gegenwart eines Inhibitors der TGF-β-Familie, eines GSK3β-Inhibitors und eines BMP-Inhibitors;
(2) Suspensionskultivierung der in Schritt (1) erhaltenen Zellen in Gegenwart eines Inhibitors der TGF-β-Familie, eines GSK3β-Inhibitors, FGF8 und eines Hedgehog-Signalagonisten und unter normalem Sauerstoffpartialdruck zur Bildung einer Neurosphäre; und
(3) Sammeln der Zellen, die die Neurosphäre bilden, um deren Differenzierung zu Dopamin-Neuronen zu induzieren, oder direkte Induktion der Neurosphäre zu Dopamin-Neuronen.

13. Verfahren zur Bewertung der Qualität einer Zellpopulation, das ein zellbasiertes Assaysystem verwendet, um ein Bewegungsmuster zu erhalten, das durch die Richtungsbewegungsfähigkeit einzelner Zellen, die eine Zellpopulation bilden, als Index definiert ist, wobei die Zellen Neuronen sind, die von induzierten pluripotenten Stammzellen stammen.

14. Verwendung eines automatischen Erkennungssystems zur automatischen Erkennung und/oder Quantifizierung von Bewegungsmustern im Verfahren nach einem der Ansprüche 1 bis 13.

## Revendications

1. Procédé d'évaluation de médicament utilisant un système de dosage à base de cellules pour obtenir un motif de déplacement défini par une capacité de déplacement directionnel de cellules individuelles constituant une population cellulaire en tant qu'indicateur, dans lequel les cellules sont des neurones dérivés de cellules souches pluripotentes induites.

2. Procédé selon la revendication 1, comprenant les étapes suivantes consistant à :
(i) préparer une population de cellules présentant une capacité de déplacement, commencer une culture en présence d'une substance de test, puis mesurer des positions des cellules individuelles qui se sont déplacées au fil du temps,
(ii) analyser la capacité de déplacement directionnel des cellules respectives à partir des informations sur les positions mesurées, totaliser les résultats d'analyse, et déterminer un motif de déplacement de la population cellulaire, et
(iii) déterminer une efficacité ou une toxicité de la substance de test sur la base du motif de déplacement déterminé.

3. Procédé selon la revendication 2, dans lequel un rapport de distance calculé dans le point (A) suivant, un angle θ déterminé dans le point (B) suivant, ou une vitesse calculée dans le point (C) suivant est utilisé(e) pour l'analyse de l'étape (ii) :
(A) calculer le rapport de distance d(t)/D(t), où D(t) est une distance de déplacement totale de chacune des cellules à un temps de mesure t et d(t) est une distance linéaire entre deux points, c'est-à-dire un point de début de déplacement et un point de fin de déplacement,
(B) définir un intervalle de temps de mesure t/n, où n est un entier de 2 ou plus, par rapport au temps de mesure t pour mesurer une position de chacune des cellules, en déterminant ainsi l'angle θ entre un vecteur de position de chacune des cellules et un axe de référence à chaque point de temps, et
(C) calculer la vitesse D(t)/t, où D(t) est la distance de déplacement totale de chacune des cellules au temps de mesure t.

4. Procédé selon la revendication 3, dans lequel un vecteur de position moyen calculé à partir d'une trajectoire de déplacement de chacune des cellules jusqu'au temps t est utilisé comme axe de référence de (B).

5. Procédé selon l'une quelconque des revendications 2 à 4, dans lequel les cellules de l'étape (i) sont des cellules anormales, et à l'étape (iii), l'efficacité de la substance de test est déterminée en utilisant une normalisation du motif de déplacement en tant qu'indicateur.

6. Procédé selon l'une quelconque des revendications 2 à 4, dans lequel les cellules de l'étape (i) sont des cellules normales, et à l'étape (iii), la toxicité de la substance de test est déterminée en utilisant une anomalie du motif de déplacement en tant qu'indicateur.

7. Procédé selon l'une quelconque des revendications 2 à 4, dans lequel les cellules de l'étape (i) sont des cellules anormales, et à l'étape (iii), la toxicité de la substance de test est déterminée en utilisant une autre anomalie du motif de déplacement en tant qu'indicateur.

8. Procédé selon la revendication 5 ou 7, dans lequel les cellules anormales sont des cellules malades présentant des caractéristiques génétiques d'une maladie cible.

9. Procédé selon la revendication 8, dans lequel les cellules malades sont des cellules provenant du patient ou des cellules préparées par génie génétique.

10. Procédé selon la revendication 9, dans lequel les cellules provenant du patient sont des cellules obtenues en induisant une différenciation de cellules souches pluripotentes induites préparées à partir de cellules de patient.

11. Procédé selon l'une quelconque des revendications 1 à 10, dans lequel les neurones sont des neurones dopaminergiques, des neurones glutamatergiques, des neurones sérotoninergiques ou des neurones GABAergiques.

12. Procédé selon la revendication 11, dans lequel les neurones dopaminergiques sont préparés par le procédé comprenant les étapes (1) à (3) suivantes consistant à :
(1) cultiver des cellules souches pluripotentes en présence d'un inhibiteur de la famille TGF-β, d'un inhibiteur de GSK3β et d'un inhibiteur de BMP ;
(2) cultiver en suspension les cellules obtenues à l'étape (1) en présence d'un inhibiteur de la famille TGF-β, d'un inhibiteur de GSK3β, de FGF8 et d'un agoniste de signal Hedgehog et sous une pression partielle d'oxygène normale pour former une neurosphère ; et
(3) collecter les cellules constituant la neurosphère pour induire leur différenciation en neurones dopaminergiques, ou induire directement la neurosphère en neurones dopaminergiques.

13. Procédé pour évaluer une qualité d'une population cellulaire en utilisant un système de dosage à base de cellules pour obtenir un motif de déplacement défini par une capacité de déplacement directionnel de cellules individuelles constituant une population cellulaire en tant qu'indicateur, dans lequel les cellules sont des neurones dérivés de cellules souches pluripotentes induites.

14. Utilisation d'un système de détection automatique pour détecter et/ou quantifier automatiquement des motifs de déplacement dans le procédé selon l'une quelconque des revendications 1 à 13.
